(19) Europäisches Patentamt

European Patent Office

Office européen des brevets

(11) Veröffentlichungsnummer: **0 250 884 B1**

(12) **EUROPÄISCHE PATENTSCHRIFT**

(45) Veröffentlichungstag der Patentschrift: **19.05.93**

(21) Anmeldenummer: **87107802.8**

(22) Anmeldetag: **29.05.87**

(51) Int. Cl.⁵: **C12M  1/14**

(54) **Durchführung von Biokatalysator−reaktionen in einem Wirbelbett−reaktor mit flüssigem 2 Phasen−System.**

(30) Priorität: **02.06.86 DE 3618465**

(43) Veröffentlichungstag der Anmeldung:
**07.01.88 Patentblatt  88/01**

(45) Bekanntmachung des Hinweises auf die
Patenterteilung:
**19.05.93 Patentblatt  93/20**

(84) Benannte Vertragsstaaten:
**AT BE CH DE FR GB IT LI NL SE**

(56) Entgegenhaltungen:
**WO−A−82/01563**
**FR−A− 1 529 536**

**BIOTECHNOLOGY & BIOENGINEERING, Band 26, Nr. 2, Februar 1984, Seiten 134−141, John Wiley & Sons, Inc., New York, US; G.M. BLACK et al.: "Practical reactor systems for yeast cell immobilization using biomass support particles"**

(73) Patentinhaber: **HOECHST AKTIENGESELL−SCHAFT**
**Postfach 80 03 20**
**W−6230 Frankturt am Main 80(DE)**

(72) Erfinder: **Rothert, Reinhardt**
**Idsteiner Strasse 86**
**W−6272 Niederhausen/Taunus(DE)**
Erfinder: **Wullbrandt, Dieter, Dr.**
**Bienerstrasse 29**
**W−6238 Hofheim am Taunus(DE)**

## Beschreibung

Die Wirbelbett – Technik ist eine verbreitete Methode in der chemischen Technologie und wird über – wiegend in Trocknungsverfahren eingesetzt. Der Wirbelbettreaktor ist außerdem in der Fermentationstech – nologie, beispielsweise in Form von Blasensäulen, anzutreffen. Das Antibiotikum Patulin kann zum Beispiel in einem derartigen Reaktor mit Hilfe von immobilisiertem Penicillium urticae in guten Ausbeuten hergestellt werden [Berk et al., Can. J. Chem.Eng. 62, 120 (1984)].

In der Enzymtechnologie hat sich ebenfalls der Wirbelbettreaktor bewährt, insbesondere beim Einsatz immobilisierter Enzyme. Mit Hilfe dieser Technologie konnte die Stabilität und damit die Raum – Zeit – Ausbeute bestimmter Enzyme wesentlich erhöht werden, was daher auch zu industrieller Anwendung führte. Sofern in diesen Systemen Flüssigkeiten eingesetzt werden, bestanden diese bislang nur aus einer Phase, vornehmlich wäßrigen Lösungen. Insofern konnte man in einem derartigen System auch nur Substrate einsetzen, die weitgehend wasserlöslich sind.

Insbesondere in der Enzymtechnologie besteht jedoch der Wunsch, auch nicht – wasserlösliche Ver – bindungen als Substrate einsetzen zu können. Ein entsprechendes System wurde bisher nur in Rührreak – toren verwirklicht. Dabei befindet sich das Enzym in der wäßrigen Phase und das Substrat in einer mit Wasser nicht – mischbaren organischen Phase. Beide Phasen werden durch Rühren innig miteinander vermischt, so daß das Enzym sein Substrat in Form von feinen Tröpfchen erreichen kann. Dieses System war bislang noch nicht auf Wirbelbettreaktoren übertragbar, da das Problem der Entmischung von wäßriger und organischer Phase nicht gelöst war. Immobilisierte Biokatalysatoren können zwar auch in Rührreaktoren eingesetzt werden, man beobachtete jedoch dabei einen stärkeren Verschleiß des Immobilisats als im Wirbelbettreaktor durch verstärkten Abrieb.

Um die Vorteile des Wirbelbettreaktors auch für Reaktionen mit Biokatalysatoren in zweiphasigen, flüssigen Systemen nutzen zu können, wurde eine entsprechende Vorrichtung geschaffen, in dem die befürchtete Entmischung der beiden flüssigen Phasen überraschenderweise kein Problem mehr darstellt.

Die Erfindung betrifft somit einen Reaktor zur Durchführung von Reaktionen mit Biokatalysatoren in einem zweiphasigen, flüssigen System, der gekennzeichnet ist durch einen säulenförmigen Behälter (1), in dem sich der Biokatalysator und die zweiphasige Flüssigkeit (8) befindet und der

a) im Kopf des Gefäßes mit einem Rührer (2),

b) mit einem Kühl/Heizmantel (3) und

c) mit einer Ablaufvorrichtung mit Filtersieb (4) versehen ist, die

d) mit einer Leitung (5) verbunden ist, über die die flüssigen Phasen im Kreislauf über

e) eine Pumpe (6) und

f) eine Zulaufvorrichtung mit Filtersieb (7) wieder in den Reaktor zurückgeführt werden, wobei Behälter und Pumpe so dimensioniert sind, daß die durch die Pumpleistung vermischte zweiphasige Flüssigkeit sich auf dem Weg durch den Behälter nicht entmischt.

Die Erfindung betrifft ebenso ein Verfahren zum Umsetzen von Substraten mit Biokatalysatoren in einem zweiphasigen, flüssigen System, das dadurch gekennzeichnet ist, daß die Umsetzung in einem Wirbelbettreaktor durchgeführt wird, dessen Verhältnis Höhe zu Durchmesser 40 : 1 ist.

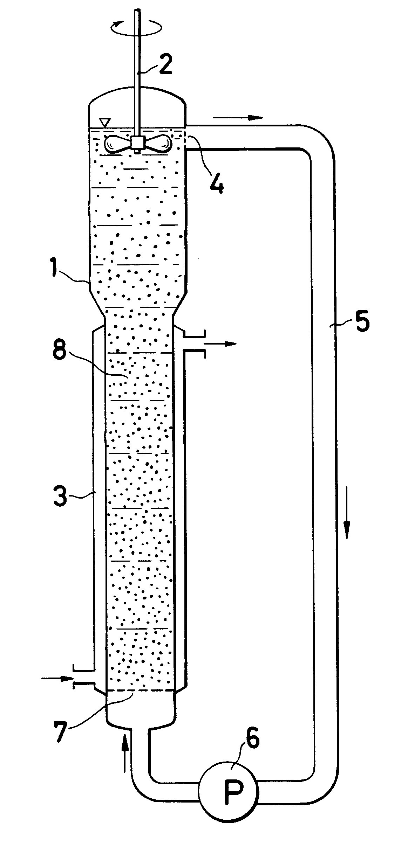

Die Erfindung wird in der Abbildung dargestellt und im folgenden genauer beschrieben, bzw. in den Patentansprüchen definiert.

Der Reaktor (1) hat Säulenform und besteht zweckmäßig aus Glas. Es können aber auch andere inerte Materialien verwendet werden. Das Verhältnis von Höhe zu Durchmesser beträgt bevorzugt 40 : 1, insbesondere bevorzugt 20 : 1. In der Säule befindet sich der Biokatalysator, zusammen mit der zweipha – sigen Flüssigkeit (8), d.h. hier findet die Umsetzung statt.

Der Rührer (2) im Säulenkopf verhindert die Ablaufverstopfung durch eventuell aufschwimmendes Katalysatormaterial und beschleunigt ferner die Sedimentation. Der säulenförmige Körper ist zur Thermo – statisierung über 1/2 bis zu 2/3 der Körperlänge mit einem Kühl – bzw. Heizmantel (3) umgeben. Die Zulaufvorrichtung (7) und insbesondere die Ablaufvorrichtung (4) ist mit Filtersieben versehen, um ein Auswaschen des Biokatalysators zu verhindern. Die Porengröße dieser Siebe richtet sich nach dem Partikeldurchmesser des Trägers und kann daher in weiten Bereichen schwanken. Verwendet man immo – bilisierte Biokatalysatoren, so setzt man Filtersiebe ein, die eine Durchlässigkeit von 50 bis 500 $\mu$m, vorzugsweise 90 bis 250$\mu$m, haben. Es können alle inerten Siebmaterialien, d.h. Stoffe, die die Katalysa – toraktivität nicht wesentlich beeinflussen, verwendet werden. Vorzugsweise werden Kunststoff oder Metall – siebe eingesetzt. Um einen ungehinderten Ablauf zu gewährleisten, sollte der entsprechende Ausgangs – querschnitt zweckmäßig etwa 10 % größer sein als die Zulauffläche.

Die Zulaufvorrichtung ist mit einer Pumpe (6) versehen, mit deren Hilfe die wäßrige und organische Phase durch die Säule und über eine Leitung (5), die aus einem Material bestehen muß, daß von den flüssigen Phasen nicht angegriffen werden kann, im Kreis zurück in die Säule gepumpt werden. Infolge der Pumptätigkeit werden die beiden Phasen innig miteinander vermischt und der in der Säule befindliche Biokatalysator fluidisiert. Die Pumpleistung muß mindestens so eingestellt sein, daß eine Durchmischung der beiden flüssigen Phasen in dem gesamten Säulenvolumen und dadurch der Biokatalysator/Substrat Kontakt gewährleistet ist. Vorzugsweise wird mit einem etwa 1,5 fachen Bettvolumen−Wechsel/min. gearbeitet. Verdrängungspumpen, insbesondere Pulsationspumpen werden bevorzugt eingesetzt.

Die organische Phase ist mit Wasser nicht oder nur schwer mischbar. Geeignet sind unter dieser Voraussetzung alle Lösungsmittel, die den Biokatalysator in seiner Aktivität nicht negativ beeinflussen.

Der erfindungsgemäße Reaktor kann vorteilhaft für alle Reaktionen mit Biokatalysatoren in zweiphasigen, flüssigen Systemen eingesetzt werden. Unter dem Begriff Biokatalysator sind Enzyme oder Zellen zu verstehen, die sowohl in freier als auch in immobilisierter Form eingesetzt werden können. Werden leichte Biokatalysatoren verwendet, deren Dichte ähnlich der eingesetzten flüssigen Phase ist, so ist es vorteilhaft, statische Mischer als Strömungsstabilisatoren einzubauen.

Im folgenden wird anhand von Beispielen die Anwendung des erfindungsgemäßen Reaktors näher erläutert. Prozentangaben beziehen sich auf das Gewicht, sofern nicht anders angegeben.

Beispiel 1:

In dem erfindungsgemäßen Glas−Wirbelbettreaktor mit einer Höhe von 60 cm und einem Durchmesser von 2,0 cm, der gemäß der Abbildung durch eine PTFE−Leitung mit 5 mm Durchmesser über eine mit Metallgewebe (1.4301, Durchlässigkeit 200 $\mu$m) versehene Zulauf− bzw. Ablaufvorrichtung mit einer Pumpe verbunden ist, werden 15 g immobilisiertes $\alpha$−Chymotrypsin, das analog Beispiel 1 der Europäischen Offenlegungsschrift 01 78 553 hergestellt wurde, in 430 ml mit Methylisobutylketon gesättigtem Wasser suspendiert.

Nach Erreichen einer stabilen Dispersion bei einer Temperatur von 30°C und einer Umpumprate von 30 l/h wird die Reaktion durch Zugabe einer Lösung aus 15 g D,L−2−(4−Hydroxyxyphenoxy)− propionsäureethylester in 20 ml Methylisobutylketon gestartet.

Der pH−Wert der Reaktionslösung von 6,5 wird durch Zugabe von 1 normaler Natronlauge in das Reaktionsgefäß mit Hilfe einer Elektrode und einer automatischen Bürette, die sich im Ablauf befindet, nachreguliert.

Nach ungefähr 8 Stunden, bei Erreichen von 30 % Umsatz bezüglich des eingesetzten Esters, der anhand des Laugenverbrauchs bestimmt wird, wird die 2−phasige Reaktionslösung wie folgt aufgearbeitet:

Die Lösung, bestehend aus L−2−(4−Hydroxyphenoxy)propionsäureethylester und dem verbleibenden D−Ester sowie der entsprechenden D−Säure, wird mit Methylisobutylketon extrahiert. Die über $Na_2SO_4$ getrocknete organische Phase wird eingeengt und der Rückstand im Vakuum bei 0,05 Torr und 135°C destilliert, racemisiert und zur weiteren Racematspaltung wieder zurückgeführt.

Die wäßrige Phase wird mit Salzsäure auf einen pH−Wert von 1−2 eingestellt und mit tert.− Butylmethylether extrahiert. Die über $Na_2SO_4$ getrocknete Etherphase wird eingeengt und zur Kristallisation der D−2−(4−Hydroxyphenoxy)propionsäure mit Petrolether versetzt.

Für das immobilisierte Enzym konnte mit dem Racemat des 2−(4−Hydroxyphenoxy)− propionsäureethylesters als Substrat eine Anfangsaktivität von 3 Units/g bestimmt werden. Zur Bestimmung der Stabilität des Biokatalysators wurden in der beschriebenen Versuchsapparatur mehrere Läufe durchgeführt. Die ermittelten Enzymaktivitäten sind in nachfolgender Tabelle (Beispiel 2) aufgelistet.

Beispiel 2:

In einem Rührreaktor, versehen mit pH−Elektrode und automatischer Bürette werden 8 g D,L−2−(4− Hydroxyphenoxy)propionsäureethylester, gelöst in 20 ml Methylisobutylketon, gegeben und in 240 ml 0,1 molarem Phosphatpuffer (pH 6,5) dispergiert. Durch Zugabe von 8 g immobilisiertem $\alpha$−Chymotrypsin wird die Reaktion gestartet und der pH−Wert von 6,5 durch Zugabe von 1 normaler Natronlauge nachreguliert. Nach ca. 24 Stunden sind 30 % des Racemats gespalten.

Nach der Aufarbeitung der Reaktionslösung analog Beispiel 1 wurde das immobilisierte Enzym wiederholt unter den obigen Bedingungen für die Racematspaltung eingesetzt. Die ermittelten Enzymaktivitäten sind in folgender Tabelle mit den in Beispiel 1 gefundenen Werten vergleichend aufgelistet.

EP 0 250 884 B1

| Batchläufe | Units/g Träger | |
|---|---|---|
| | Wirbelbettreaktor (Beispiel 1) | Rührreaktor (Beispiel 2) |
| 1 | 2,9 | 1,5 |
| 2 | 2,9 | 1,43 |
| 3 | 2,2 | 1,49 |
| 4 | 2,9 | 1,20 |
| 5 | 2,85 | 1,27 |
| 6 | 2,8 | 1,21 |
| 7 | 3,0 | 1,01 |
| 8 | 3,0 | 1,20 |
| 9 | 3,2 | 0,91 |
| 10 | 3,2 | 1,12 |

Die optische Reinheit der aus Beispiel 1 und 2 gewonnenen D – 2(4 – Hydroxyphenoxy)propionsäure wurde mit 95 % Enantiomeren – Überschuß bestimmt: $[\alpha]_D^{20}$ + 42,3° (c = 1 in Ethanol)

Eine Abnahme der Enzymselektivität konnte im Verlauf der Zeit nicht beobachtet werden.

Beispiel 3:

Man verfährt nach Beispiel 1, dosiert jedoch kontinuierlich mit einer Rate von 45 ml/h eine Dispersion aus 1000 ml Wasser und 60 g D,L – 2(4 – Hydroxyphenoxy)propionsäureethylester, gelöst in 80 ml Methy – lisobutylketon, zu, so daß sich im System ein Umsatz von 30 % einstellt. Diese kontinuierliche Verfahrens – weise konnte über einen Zeitraum von 200 Stunden ohne merkliche Abnahme der Enzymaktivität betrieben werden.

**Patentansprüche**

1. Reaktor zur Durchführung von Reaktionen mit Biokatalysatoren in einem zweiphasigen, flüssigen System, gekennzeichnet durch einen säulenförmigen Behälter (1), in dem sich der Biokatalysator und die zweiphasige Flüssigkeit (8) befindet und der
   a) im Kopf des Behälters mit einem Rührer (2),
   b) mit einem Kühl – /Heizmantel (3) und
   c) mit einer Ablaufvorrichtung mit Filtersieb (4) versehen ist, die
   d) mit einer Leitung (5) verbunden ist, über die die flüssigen Phasen im Kreislauf über
   e) eine Pumpe (6) und
   f) eine Zulaufvorrichtung mit Filtersieb (7) wieder in den Reaktor zurückgeführt werden, wobei Behälter und Pumpe so dimensioniert sind, daß die durch die Pumpleistung vermischte zweiphasige Flüssigkeit sich auf dem Weg durch den Behälter nicht entmischt.

2. Reaktor nach Anspruch 1, dadurch gekennzeichnet, daß das Verhältnis Höhe zu Durchmesser des säulenförmigen Behälters 40 : 1 ist.

3. Reaktor nach Anspruch 2, dadurch gekennzeichnet, daß das Verhältnis Höhe zu Durchmesser des säulenförmigen Behälters 20 : 1 ist.

4. Verfahren zum Umsetzen von Substraten mit Biokatalysatoren in einem zweiphasigen, flüssigen System, dadurch gekennzeichnet, daß die Umsetzung in einem Wirbelbettreaktor gemäß Anspruch 1 durchgeführt wird, dessen Verhältnis Höhe zu Durchmesser 40 : 1 ist.

4

## EP 0 250 884 B1

**Claims**

1. A reactor for carrying out reactions with a biocatalyst in a two – phase liquid system, which comprises a columnar vessel (1) which contains the biocatalyst and the two – phase liquid (8) and which is provided
   a) with a stirrer (2) in the top of the vessel,
   b) with a cooling/heating jacket (3) and
   c) with a discharge device containing a filter screen (4) and connected
   d) to a line (5), through which the liquid phases are recirculated into the reactor via
   e) a pump (6) and
   f) a feed device with a filter screen (7), the vessel and the pump being dimensioned such that the two – phase liquid mixed by the pumping energy does not segregate on passing through the vessel.

2. The reactor as claimed in claim 1, wherein the height/diameter ratio of the columnar vessel is 40:1.

3. The reactor as claimed in claim 2, wherein the height/diameter ratio of the columnar vessel is 20:1.

4. A process for reacting substrates with biocatalysts in a two – phase liquid system, which comprises carrying out the reaction in a fluidized – bed reactor as claimed in claim 1 having a height/diameter ratio of 40:1.

**Revendications**

1. Réacteur pour effectuer des réactions avec des biocatalyseurs dans un système liquide à deux phases, caractérisé par un récipient (1) en forme de colonne, dans lequel se trouvent le biocatalyseur et le liquide diphasique (8) et qui est pourvu
   a) d'un agitateur (2) en tête de récipient,
   b) d'une chemise de refroidissement/chauffage (3) et
   c) d'un dispositif d'évacuation avec tamis filtrant (4),
   d) communiquant avec un conduit (5) par lequel les phases liquides sont recyclées dans le réacteur par l'intermédiaire
   e) d'une pompe (6) et
   f) d'un dispositif d'admission avec tamis filtrant (7), le récipient et la pompe ayant des dimensions telles que le liquide diphasique mélangé par l'effet de la pompe ne subit pas de démixtion lors de son trajet à travers le récipient.

2. Réacteur selon la revendication 1, caractérisé en ce que le rapport de la hauteur au diamètre du récipient en forme de colonne vaut 40:1.

3. Réacteur selon la revendication 2, caractérisé en ce que le rapport de la hauteur au diamètre du récipient en forme de colonne vaut 20:1.

4. Procédé pour faire réagir des substrats avec des biocatalyseurs dans un système liquide à deux phases, caractérisé en ce que la réaction est effectuée dans un réacteur à lit fluidisé selon la revendication 1, dont le rapport de la hauteur au diamètre vaut 40:1.